# EUROPEAN PATENT APPLICATION

(11) **EP 4 085 880 A1**
(43) Date of publication of application: **09.11.2022**
(21) Application number: 22171208.6
(22) Date of filing: 02.05.2022
(51) Int. Cl.: A61F 5/08

(54) **NASAL DILATOR**

(30) Priority: 02.05.2021 US 202163201500 P
(71) Applicant: Corbett Lair, Inc., Sarasota FL 34235 (US)
(72) Inventor: IERULLI, Joseph, Sarasota, 34235 (US)
(74) Representative: Dickerson, David

(57) **Abstract**

A nasal dilator (500), comprising a plurality of laminated components (510, 520A, 520B, 520C, 530), wherein each of the laminated components comprises a first major surface that faces in a first direction and a second major surface that faces in a second direction, the plurality of laminated components comprises a first component and a second component, the first component comprises a first region of a first color, the second component comprises a second region, the second region is a semi-transparent region of a second color, and the second region overlaps the first region such that the first color is visible via the second region and the second color alters a visual perception of the first color.

## Description

### CROSS-REFERENCE TO RELATED APPLICATIONS

This application claims priority under 35 U.S.C. § 119 to U.S. Provisional Application No. 63/201,500, filed on May 2, 2021, the entire disclosure of which is herein expressly incorporated by reference.

### BACKGROUND OF THE DISCLOSURE

### FIELD OF THE DISCLOSURE

The present disclosure relates to a nasal dilator as well as to a nasal dilator manufacture method.

### DESCRIPTION OF THE RELATED ART

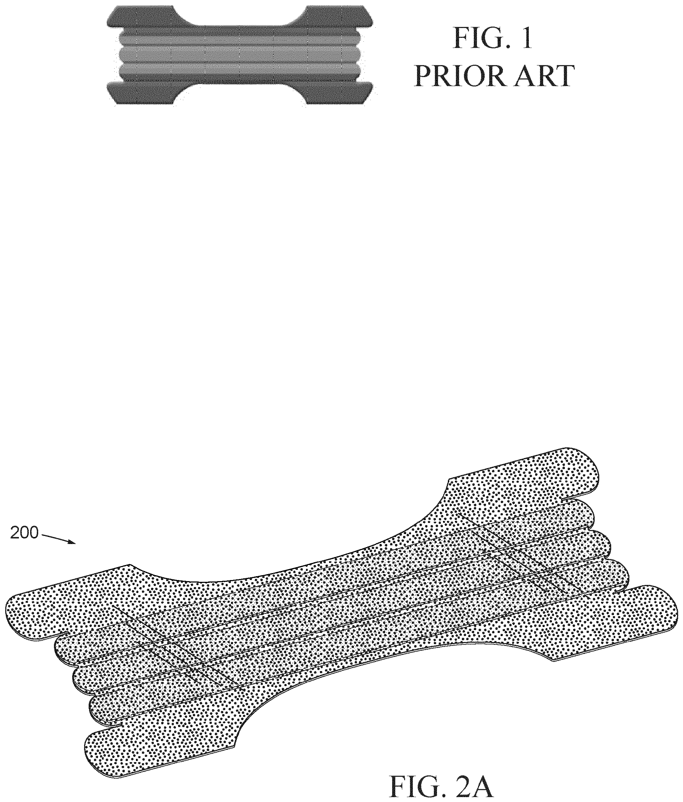

A wide variety of nasal dilators are known. Figure 1, for example, shows a nasal dilator with a multi-colored pattern imprinted on a cover layer of the nasal dilator.

The present disclosure expounds upon this background.

### SUMMARY OF THE PRESENT DISCLOSURE

The aim of the present summary is to facilitate understanding of the present disclosure. The summary thus presents concepts and features of the present disclosure in a more simplified form and in looser terms than the detailed description below and should not be taken as limiting other portions of the present disclosure.

Loosely speaking, the present disclosure discloses a nasal dilator comprising a plurality of laminated components. One of the laminated components has a first region of a first color and another of the laminated components has a semi-transparent region of a second color. The semi-transparent region overlaps the first region such that first color is visible via the colored semi-transparent region and the second color alters a visual perception of the first color. The use of a colored semi-transparent region to provide a multi-color effect allows for a greater diversity of colors and/or can simplify color alignment (known in the art of printing as "registration") without adversely affecting the manufacturability / manufacturing cost of the multi-color nasal dilator.

Other objects, advantages and embodiments of the present disclosure will become apparent from the detailed description below, especially when considered in conjunction with the accompanying drawings.

### BRIEF DESCRIPTION OF THE DRAWINGS

The Figures show:
- Fig. 1: a depiction of a nasal dilator of the prior art.
- Fig. 2A: a schematic depiction of a nasal dilator in accordance with the present disclosure.
- Fig. 2B: an exploded view schematically depicting the nasal dilator of Fig. 2A.
- Fig. 2C: a schematic depiction of the nasal dilator of Fig. 2A in use.
- Fig. 3A: a schematic depiction of a nasal dilator in accordance with the present disclosure.
- Fig. 3B: an exploded view schematically depicting the nasal dilator of Fig. 3A.
- Fig. 4: a schematic depiction of a model of subtractive color mixing
- Fig. 5A: a schematic depiction of a nasal dilator in accordance with the present disclosure.
- Fig. 5B: an exploded view schematically depicting the nasal dilator of Fig. 5A.
- Fig. 6A: an exploded view schematically depicting a nasal dilator in accordance with the present disclosure.
- Fig. 6B: a schematic depiction of the nasal dilator of Fig. 6A.
- Fig. 7A: an exploded view schematically depicting a nasal dilator in accordance with the present disclosure.
- Fig. 7B: a schematic depiction of the nasal dilator of Fig. 7A.
- Fig. 8: a schematic depiction of a nasal dilator in accordance with the present disclosure.

### DETAILED DESCRIPTION

The various embodiments of the present disclosure and of the claimed invention, in terms of both structure and operation, will be best understood from the following detailed description, especially when considered in conjunction with the accompanying drawings.

Before elucidating the embodiments shown in the Figures, the various embodiments of the present disclosure will first be described in general terms.

The present disclosure discloses a(n external) nasal dilator, *e.g.* a nasal dilator configured to be flexed over the bridge of a user's nose to dilate a nasal passage.

The nasal dilator may comprise a plurality of components. Each of the components may be affixed - *e*.*g*. (adhesively) adhered, (ultrasonically, thermally, and/or adhesively) bonded, pressure bonded, or stitched - to at least one other component of the plurality of components. The plurality of components may form a plurality of layers, each layer comprising / consisting of at least one of the plurality of components. As such, the plurality of components may be termed a plurality of laminated components or simply a laminate. In a state of use, the laminate may constitute (an entirety of) the nasal dilator.

Each of the components may comprise a first major surface that faces in a first direction and a second major surface that faces in a second direction (opposite / anti-parallel to the first direction). Similarly, the laminate, *i*.*e*. the (overall) plurality of (laminated) components, may comprise a first outermost surface that faces in the first direction and a second outermost surface that faces in the second direction. The first outermost surface may define a first side of the laminate, and the second outermost surface may define a second side of the laminate. In use, the first outermost surface may constitute an outermost surface on a first side of the nasal dilator, and the second outermost surface may constitute an outermost surface on a second side of the nasal dilator.

At least 10%, at least 20%, at least 50%, at least 80%, or at least 90% (of a total area) of the first outermost surface of the laminate may be coated with a (biocompatible) adhesive, e.g. for (releasably) affixing the nasal dilator to a user's skin. The adhesive may be a pressure-sensitive adhesive.

The nasal dilator may comprise a (sheet-like) release liner that, e.g. during transport and/or storage of the nasal dilator, covers the adhesive on the first outermost surface of the laminate. The release liner may be releasably adhered to the laminate (via the adhesive). The release liner may be manually removable from the laminate, e.g. to expose the adhesive (immediately prior to affixing the nasal dilator to a user's skin). In the present specification, the term "manually" may be understood as meaning using (no other tool than) human hands. The presence of the release liner may be an indication of a (partially) protected state of the nasal dilator. As touched upon above, the absence / removal of the release liner may be a prerequisite for use of the nasal dilator, *i*.*e*. for the nasal dilator to be in a state of use. The release liner may cover an entire area of the first outermost surface of the laminate or at least 60%, at least 80%, at least 90%, or at least 95% of an entire area of the first outermost surface of the laminate. The release liner may comprise a substrate of a (Kraft) paper and/or a synthetic material, *e.g.* a plastic film. At least one major surface of the substrate may be coated with a release agent, *e.g.* silicone, or other coating. The release liner may constitute an outermost component (on the first side) of the nasal dilator (in a (partially) protected state).

The nasal dilator may comprise a (manually removable) cover liner. The cover liner may be (manually removably) affixed, e.g. via an adhesive, to the second outermost surface of the laminate. The presence of the cover liner may be an indication of a (partially) protected state of the nasal dilator. As touched upon above, the absence / removal of the cover liner may be a prerequisite for use of the nasal dilator, *i.e.* for the nasal dilator to be in a state of use. The cover liner may cover an entire area of the second outermost surface of the laminate or at least 60%, at least 80%, at least 90%, or at least 95% of an entire area of the second outermost surface of the laminate. The cover liner may comprise a sheet-like material such as (Kraft) paper and/or plastic film. The cover liner may constitute an outermost component (on the second side) of the nasal dilator (in a (partially) protected state).

The plurality of components may comprise at least one resilient member. For example, the nasal dilator may comprise one, two or three resilient member(s). In the present disclosure, the term "resilient member" may be understood as a component of the nasal dilator configured to provide therapeutic function by dilating an anatomical structure of a user, in particular a nasal passage. More specifically, the term "resilient member" may be understood as a component configured to be flexed over the bridge of a user's nose to (provide return forces that) dilate a nasal passage. An entirety or at least a portion of (the second major surface of) any of the at least one resilient member may be white or other light color. *(An elucidation of the term "any" is given in the closing paragraphs of this specification.)* In the present disclosure, a "light color" may be understood as a color for which none of the four halftone percentage values representing that (light) color in CMYK color space exceeds 10. An entirety or at least a portion of any of the at least one resilient member may be opaque. An entirety or at least a portion of any of the at least one resilient member may be transparent, semi-transparent, or translucent.

The plurality of components may comprise at least one base layer. For example, the nasal dilator may comprise one, two or three base layer(s). An entirety or at least a portion of (the second major surface of) any of the at least one base layer may be white or other light color. An entirety or at least a portion of any of the at least one base layer may be opaque. The first major surface of at least one base layer may constitute (an entirety of / at least a corresponding portion of) the first outermost surface. The second major surface of at least one base layer may face (the first major surface of) at least one of the at least one resilient member. Thus, in the present disclosure, the term "base layer" may be understood as a component of the nasal dilator, wherein the first major surface of said component constitutes at least a portion of the first outermost surface and wherein the second major surface of said component faces at least one of the at least one resilient member. In the present disclosure, the term "base layer" may be understood as a component of the nasal dilator configured to (directly / indirectly) interconnect at least one of the at least one resilient member with a user's skin. In the present disclosure, the term "base layer" may be understood as a component of the nasal dilator comprising an (manually) exposable adhesive on the first major surface of said component and wherein the second major surface is (directly) affixed to (the first major surface of) at least one of the at least one resilient member or (directly) affixed to an interface member, e.g. as described below, which interface member is (directly) affixed to (the first major surface of) at least one of the at least one resilient member.

The plurality of components may comprise at least one cover layer. For example, the nasal dilator may comprise one, two or three cover layer(s). The at least one cover layer may constitute (an entirety of / at least a corresponding portion of) the second outermost surface. The first major surface of at least one cover layer may face (the second major surface of) at least one of the at least one resilient member. In the present disclosure, the term "cover layer" may be understood as a component of the nasal dilator, wherein the first major surface of said component is (directly) affixed to (the second major surface of) at least one of the at least one resilient member. In the present disclosure, the term "cover layer" may be understood as a component of the nasal dilator, wherein the first major surface of said component is (directly) affixed to (the second major surface of) at least one of the at least one base layer and, wherein the second major surface of said component constitutes (an entirety of / at least a corresponding portion of) the second outermost surface.

The plurality of components may comprise at least one interface member. For example, the nasal dilator may comprise one, two or three interface member(s). An entirety or at least a portion of (the second major surface of) any of the at least one interface member may be white or other light color. An entirety or at least a portion of any of the at least one interface member may be opaque. In the present disclosure, the term "interface member" may be understood as a component of the nasal dilator situated between at least one of the at least one base layer and at least one of the at least one resilient member. In the present disclosure, the term "interface member" may be understood as a component of the nasal dilator, wherein the first major surface of said component is (directly) affixed to (the second major surface of) at least one of the at least one base layer and the second major surface of said component is (directly) affixed to (the first major surface of) at least one of the at least one resilient member. The interface member may be of a soft (relative to human skin) material and/or of a supple (relative to human skin) material. The interface member may comprise / be of a foam material. Any (respective individual interface member) of the at least one interface member may have a peripheral shape that matches / corresponds to a peripheral shape of any (respective individual resilient member) of the at least one resilient member.

The plurality of components may comprise a first component. The first component may comprise a first region of a first color. The first region may be an entirety of the first component, and the entirety of the first component may be of the first color, e.g. of a (synthetic or natural) material of the first color. Additionally or alternatively, a(n entirety) of the second major surface of the first component or a portion of the second major surface may constitute the first region and may comprise a coating and/or an overlay of the first color. The portion / entirety of the second major surface may be printed and/or dyed with ink of the first color. For example, the portion / entirety of second major surface may be coated with a layer of ink and/or paint of the first color and/or be overlaid with a sheet of material (having a (painted / dyed) surface) of the first color.

The first color may be a solid color. An entirety of the first region may be monochromatic and of the first color. Alternatively, a(n entirety) of the second major surface of the first component or a portion of the second major surface may comprise a polychromatic coating and/or a polychromatic overlay. The polychromatic coating / overlay may comprise a metallic paint, a pearlescent paint, or a combination materials that provide a "candy apple" effect. The polychromatic coating / overlay may be a polychromatic coating / overlay obtainable using a single batch of metallic / pearlescent paint. Similarly, (the (entirety) of the second major surface of) the first component may be of or comprise a thermochromic substance and/or a photochromic substance. As such, the first color may be a color of a polychromatic coating / overlay. Similarly, the first color may be a (transient) color resulting, at least in part, from a thermochromic and/or photochromic substance, *i*.*e*. a (transient) color to which the thermochromic and/or photochromic substance contributes.

In the present disclosure, the term "color" may be understood as a (humanly, visually) perceptible color, not excluding black, white, or any shade of gray. Similarly, the term "color" may be understood in the present disclosure as a (humanly, visually) perceptible polychromatic appearance, not excluding (constituents of) black, white, and/or any shades of gray. Alternatively, the term "color" may be understood in the present disclosure as a (humanly, visually) perceptible color, excepting black, white, and all shades of gray. Similarly, the term "color" may be understood in the present disclosure as a (humanly, visually) perceptible polychromatic appearance, excluding (constituents of) black, white, and any shades of gray.

The plurality of components may comprise a second component. The second component may comprise a second, semi-transparent region of a second (solid) color. In the present disclosure, the term "semi-transparent" may be understood as allowing (visible) light to pass, albeit with (humanly) visually perceptible effect on the characteristics of light passing through (the region / material). In the present disclosure, the term "semi-transparent" may be synonymous with the term "translucent" or may encompass the scope of the term "translucent". As such, the term "semi-transparent" may be understood (exclusively / additionally) as allowing (visible) light to pass, yet (humanly) visually perceptibly obscuring details (of scenes conveyed by the light passing through (the region / material)), *e.g.* by diffusing the light passing through (the region / material).

For example, the second component may be / comprise a plastic film, e.g. a plastic film constituting one of the at least one cover layer. An entirety of the plastic film may comprise coloring of the second (solid) color, and the entirety of the plastic film may constitute the second region. Similarly, a first portion of the plastic film may comprise coloring of the second (solid) color, and the first portion of the plastic film may constitute the second region. For example, a portion / entirety of the plastic film may be a semi-transparent plastic film of the second (solid) color or be semi-transparently (solidly) colored with an ink / dye / coating of the second color. a(n entirety) of the first / second major surface of the plastic film or a portion of the first / second major surface of the plastic film may constitute the first portion of the plastic film and/or constitute the second region. For example, a(n entirety) of the first / second major surface of the plastic film or a portion of the first / second major surface may be semi-transparently printed and/or dyed with ink of the second color. Similarly, a(n entirety) of the first / second major surface of the plastic film or a portion of the first / second major surface may be semi-transparently coated with a layer of ink and/or paint of the second color.

The second color may be a solid color. An entirety of the second region may be monochromatic and of the second color Alternatively, a(n entirety) of the first / second major surface of the plastic film or a portion of the first / second major surface may comprise a semi-transparent polychromatic coating. The semi-transparent polychromatic coating may comprise a metallic paint, a pearlescent paint, or a combination of materials that provide a "candy apple" effect. The polychromatic coating may be a polychromatic coating obtainable using a single batch of metallic / pearlescent paint. Similarly, a portion / entirety of the plastic film may be treated / colored with a semi-transparent thermochromic substance and/or a semi-transparent photochromic substance. As such, the second "color" of the second, semi-transparent region may be polychromatic. Similarly, the "color" of the second, semi-transparent region may be a (transient) color resulting, at least in part, from a thermochromic and/or photochromic substance, *i.e.* a (transient) color to which the thermochromic and/or photochromic substance contributes.

The second region may overlap the first region. An entirety or at least a portion of the second region may be / constitute a visibly exposed surface of the nasal dilator, e.g. when the nasal dilator is viewed in the first direction onto the second side of the nasal dilator (in use). Similarly, an entirety or at least a portion of the second region may be / constitute an entirety or a portion of the second outermost surface. An entirety or at least a portion of the first region may be visible, e.g. when the nasal dilator is viewed in the first direction onto the second side of the nasal dilator (in use), via (at least a portion of) the second region. The second color (of the second region) may alter a visual perception of the first color (of the first region). More specifically, the second region may overlap the first region such that the first color (of the first region) is visible via the second region and the second color (of the second region) alters a visual perception of the first color (of the first region). The visual perception of the first color (of the first region) visible via the second region may be (humanly) visibly perceptibly different from the first color (of the first region) *per* se, *i*.*e*. from the (actual) first color (of the first region). Furthermore, the visual perception of the first color (of the first region) visible via the second region may be (humanly) visibly perceptibly different from the second color (of the second region).

The first component may comprise a third region of a third (solid) color, e.g. in a manner analogous to the first region / first color. Similarly, the second component may comprise a fourth region, e.g. a fourth, transparent region, a fourth, semi-transparent region of a fourth (solid) color, or a fourth, opaque region of a fourth (solid) color. For example, a second portion of the plastic film may be (opaque and) of a fourth (solid) color, e.g. in a (monochromatic / polychromatic) manner analogous to the first region / first color. Similarly, the second portion may be semi-transparent and of a fourth (solid) color, e.g. in a (monochromatic / polychromatic) manner analogous to the second, semi-transparent region / second color.

The plurality of components may comprise a third component. The third component may comprise a fifth region of a fifth (solid) color, *e.g.* a fifth, semi-transparent region of a fifth (solid) color, or a fifth, opaque region of a fifth (solid) color. For example, an entirety or a portion of the third component may be (opaque and) of a fifth (solid) color, *e.g.* in a manner analogous to the first component / first region / first color. Similarly, an entirety or a portion of the third component may be semi-transparent and of a fifth (solid) color, e.g. in a (monochromatic / polychromatic) manner analogous to the second, semi-transparent region / second color.

The plurality of components may comprise a fourth component. The fourth component may comprise a sixth region of a sixth (solid) color, *e.g.* a sixth, semi-transparent region of a sixth (solid) color, or a sixth, opaque region of a sixth (solid) color. For example, an entirety or a portion of the fourth component may be (opaque and) of a sixth (solid) color, *e.g.* in a manner analogous to the first component / first region / first color. Similarly, an entirety or a portion of the fourth component may be semi-transparent and of a sixth (solid) color, e.g. in a (monochromatic / polychromatic) manner analogous to the second, semi-transparent region / second color.

An entirety or at least a portion of the fourth / fifth / sixth, semi-transparent region may be / constitute a visibly exposed surface of the nasal dilator, e.g. when the nasal dilator is viewed in the first direction onto the second side of the nasal dilator (in use). Similarly, an entirety or at least a portion of the fourth / fifth / sixth, semi-transparent region may be / constitute an entirety or a portion of the second outermost surface.

The fourth, semi-transparent region may overlap the first region. An entirety or at least a portion of the first region may be visible, *e.g.* when the nasal dilator is viewed in the first direction onto the second side of the nasal dilator (in use), via (at least a portion of) the fourth, semi-transparent region. The fourth color (of the fourth, semi-transparent region) may alter a visual perception of the first color (of the first region). More specifically, the fourth, semi-transparent region may overlap the first region such that the first color (of the first region) is visible via the fourth, semi-transparent region and the fourth color (of the fourth, semi-transparent region) alters a visual perception of the first color (of the first region). The visual perception of the first color (of the first region) visible via the fourth, semi-transparent region may be (humanly) visibly perceptibly different from the first color (of the first region) *per* se, *i.e*. from the (actual) first color (of the first region). Furthermore, the visual perception of the first color (of the first region) visible via the fourth, semi-transparent region may be (humanly) visibly perceptibly different from the fourth color (of the fourth, semi-transparent region).

The fifth, semi-transparent region and/or the sixth, semi-transparent region may overlap the first region. An entirety or at least a portion of the first region may be visible, e.g. when the nasal dilator is viewed in the first direction onto the second side of the nasal dilator (in use), via (at least a portion of) the fifth / sixth, semi-transparent region. The fifth / sixth color (of the fifth / sixth, semi-transparent region) may alter a visual perception of the first color (of the first region). More specifically, the fifth / sixth, semi-transparent region may overlap the first region such that the first color (of the first region) is visible via the fifth / sixth, semi-transparent region and the fifth / sixth color (of the fifth / sixth, semi-transparent region) alters a visual perception of the first color (of the first region). The visual perception of the first color (of the first region) visible via the fifth / sixth, semi-transparent region may be (humanly) visibly perceptibly different from the first color (of the first region) *per* se, *i*.*e*. from the (actual) first color (of the first region). Furthermore, the visual perception of the first color (of the first region) visible via the fifth / sixth, semi-transparent region may be (humanly) visibly perceptibly different from the fifth / sixth color (of the fifth / sixth, semi-transparent region).

The fourth, semi-transparent region may overlap the third region of a third (solid) color and/or the fifth region of a fifth (solid) color and/or the sixth region of a sixth (solid) color. An entirety or at least a portion of the third / fifth / sixth region may be visible, e.g. when the nasal dilator is viewed in the first direction onto the second side of the nasal dilator (in use), via (at least a portion of) the fourth, semi-transparent region. The fourth color (of the fourth region) may alter a visual perception of the third / fifth / sixth color (of the third / fifth / sixth region). More specifically, the fourth, semi-transparent region may overlap the third / fifth / sixth region such that the third / fifth / sixth color (of the third / fifth / sixth region) is visible via the fourth, semi-transparent region and the fourth color (of the fourth, semi-transparent region) alters a visual perception of the third / fifth / sixth color (of the third / fifth / sixth region). The visual perception of the third / fifth / sixth color (of the third / fifth / sixth region) visible via the fourth, semi-transparent region may be (humanly) visibly perceptibly different from the third / fifth / sixth color (of the third / fifth / sixth region) *per* se, *i.e.* from the (actual) third / fifth / sixth color (of the third / fifth / sixth region). Furthermore, the visual perception of the third / fifth / sixth color (of the third / fifth / sixth region) visible via the fourth, semi-transparent region may be (humanly) visibly perceptibly different from the fourth color (of the fourth, semi-transparent region).

The fifth, semi-transparent region may overlap the third region of a third (solid) color and/or the fourth region of a fourth (solid) color and/or the sixth region of a sixth (solid) color. An entirety or at least a portion of the third / fourth / sixth region may be visible, *e*.*g*. when the nasal dilator is viewed in the first direction onto the second side of the nasal dilator (in use), via (at least a portion of) the fifth, semi-transparent region. The fifth color (of the fifth, semi-transparent region) may alter a visual perception of the third / fourth / sixth color (of the third / fourth / sixth region). More specifically, the fifth, semi-transparent region may overlap the third / fourth / sixth region such that the third / fourth / sixth color (of the third / fourth / sixth region) is visible via the fifth, semi-transparent region and the fifth color (of the fifth, semi-transparent region) alters a visual perception of the third / fourth / sixth color (of the third / fourth / sixth region). The visual perception of the third / fourth / sixth color (of the third / fourth / sixth region) visible via the fifth, semi-transparent region may be (humanly) visibly perceptibly different from the third / fourth / sixth color (of the third / fourth / sixth region) *per* se, *i*.*e*. from the (actual) third / fourth / sixth color (of the third / fourth / sixth region). Furthermore, the visual perception of the third / fourth / sixth color (of the third / fourth / sixth region) visible via the fifth, semi-transparent region may be (humanly) visibly perceptibly different from the fifth color (of the fifth, semi-transparent region).

The sixth, semi-transparent region may overlap the third region of a third (solid) color and/or the fourth region of a fourth (solid) color and/or the fifth region of a fifth (solid) color. An entirety or at least a portion of the third / fourth / fifth region may be visible, *e*.*g*. when the nasal dilator is viewed in the first direction onto the second side of the nasal dilator (in use), via (at least a portion of) the sixth, semi-transparent region. The sixth color (of the sixth, semi-transparent region) may alter a visual perception of the third / fourth / fifth color (of the third / fourth / fifth region). More specifically, the sixth, semi-transparent region may overlap the third / fourth / fifth region such that the third / fourth / fifth color (of the third / fourth / fifth region) is visible via the sixth, semi-transparent region and the sixth color (of the sixth, semi-transparent region) alters a visual perception of the third / fourth / fifth color (of the third / fourth / fifth region). The visual perception of the third / fourth / fifth color (of the third / fourth / fifth region) visible via the sixth, semi-transparent region may be (humanly) visibly perceptibly different from the third / fourth / fifth color (of the third / fourth / fifth region) *per* se, *i.e.* from the (actual) third / fourth / fifth color (of the third / fourth / sixth region). Furthermore, the visual perception of the third / fourth / fifth color (of the third / fourth / fifth region) visible via the sixth, semi-transparent region may be (humanly) visibly perceptibly different from the sixth color (of the sixth, semi-transparent region).

In the present disclosure, the expression "alter a visual perception" may be understood as creating / effecting a (humanly) visually perceptible difference, *e.g. vis-à-vis* what previously was and/or *vis-à-vis* what otherwise would be. For example, the visual perception of a color may be altered by altering the characteristics of a path of light to and/or from a material exhibiting said color, *i.e.* a path travelled (by the light observed as the visual perception of the color) from a light source illuminating the material to an observer. On account of the altered characteristics of the path of light, the (altered) visual perception of the color may differ from the actual color, *i*.*e*. from the color *per se, i*.*e*. from a visual perception of the color without color-altering influences (excepting ambient air) along the path of light to and/or from the material exhibiting the color. In the present disclosure, the concept of one color (of a semi-transparent region) "altering a visual perception" of another color may be understood as meaning that the other color is / becomes (humanly) visually perceptible as (not the other color *(per se),* but as) yet another color on account of the one color (of the semi-transparent region), *e*.*g*. on account of an influence of the one color (of the semi-transparent region) on the characteristics of a path of light (through the semi-transparent region) to and/or from the other color. For example, the one color (of the semi-transparent region) may filter out certain wavelengths of light passing (through the semi-transparent region) toward and/or away from the other color. In other words, the one color (of the semi-transparent region) may subtractively mix with the other color to effect a (humanly) visible perception of yet another color. The yet another color may be (humanly) visually perceptibly different from both the one color and the other color.

In the present disclosure, one color may be considered (humanly) visually perceptibly different from another color if a (first) sum of four halftone percentage values representing the one color in CMYK color space differs from a (second) sum of four halftone percentage values representing the other color in CMYK color space, *e*.*g*. by at least a minimum of 5, 8, 10, 15, or 20. As such, a (first) sum of four halftone percentage values representing the first color *(per se)* of the first region in CMYK color space may differ from a (second) sum of four halftone percentage values representing a (humanly) visibly perceived color of the first region, *i.e.* a (humanly visible perception of the first color of the first region, as visible via the second region, *i*.*e*. via the second color of the second region, in CMYK color space, *e*.*g*. by at least a minimum of 5, 8, 10, 15, or 20. For example, a shade of blue represented in CMYK color space by the four halftone percentage values C=78, M=38, Y=0, and K=14 may be considered (humanly) visually perceptibly different from another shade of blue represented in CMYK color space by the four halftone percentage values C=78, M=32, Y=0, and K=14 since the sum (78+38+0+14 = 130) of the four halftone percentage values representing the one shade of blue differs from the sum (78+32+0+14 = 124) of the four halftone percentage values representing the other shade of blue by 6, *i*.*e*. by at least a minimum of 5.

In the present disclosure, a(n actual) color *(per se)* of a component / surface comprising any of a polychromatic coating, a polychromatic overlay, a thermochromic substance, and a photochromic substance may be considered (humanly) visually perceptibly different from an altered perception of the color of that component / surface, *e*.*g*. on account of material that alters, *e*.*g*. as described above, the characteristics of a path of light to and/or from that component / surface, if the altered perception of the color of that component / surface is (humanly, visually) perceptibly different, *e*.*g*. as defined above, from the (actual) color *(per se)* of that component / surface when viewed *(e.g.* under diffuse indoor lighting of at least 500 lux, *e*.*g*. from a source of white light) along each of at least three axes that differ from one another (with respect to at least one axis or at least two axes of 3-D space) by at least 30°.

The first color may be (humanly) visually perceptibly different from the second color and/or the third color and/or the fourth color and/or the fifth color. The second color may be (humanly) visually perceptibly different from the third color and/or the fourth color and/or the fifth color. The third color may be (humanly) visually perceptibly different from the fourth color and/or the fifth color. The fourth color may be (humanly) visually perceptibly different from the fifth color. As such, a (first) sum of four halftone percentage values representing the first / second / third / fourth / fifth color in CMYK color space may differ from a (second) sum of four halftone percentage values representing another of the first / second / third / fourth / fifth color in CMYK color space, *e.g.* by at least a minimum of 5, 8, 10, 15, or 20.

The first / second / third / fourth / fifth color may be (humanly, visually) perceptibly different from a (closest) human skin color. For example, a (first) sum of four halftone percentage values representing the first / second / third / fourth / fifth color in CMYK color space may differ from a (second) sum of four halftone percentage values representing a (closest) human skin color in CMYK color space, *e.g.* by at least a minimum of 5, 8, 10, 15, or 20. In the present disclosure, the term "closest human skin color" may be understood as designating a color that is closest to the respective first / second / third / fourth / fifth color while still being a human skin color. In the present disclosure, the term "human skin color" may be understood as a color of human skin, *e.g*. as (humanly, visually, macroscopically) perceived from a distance of at least two meters. An exemplary gamut of "fair" skin colors can range from pale, tannish grey *(e.g.* 4, 3, 3, 0 in CMYK color space) through light tannish peach *(e.g.* 7, 15, 15, 0 in CMYK color space) to light brown *(e.g.* 34, 51, 56, 3 in CMYK color space). Similarly, an exemplary gamut of "dark" skin colors can range from milk chocolate brown *(e.g.* 22, 73, 91, 42 in CMYK color space) to dark brown (*e*.*g*. 45, 73, 79, 69 in CMYK color space). An exemplary gamut of "Caucasian" skin colors can range from light peach (*e*.*g*. 2, 20, 39, 0 in CMYK color space) through sandy brown *(e.g.* 6, 22, 49, 1 in CMYK color space) and sandy yellow *(e.g.* 2, 9, 40, 0 in CMYK color space) to orangish (*e*.*g*. 1, 34, 60, 0 in CMYK color space). An exemplary gamut of "Brown" skin colors can range from yellowish tan *(e.g.* 9, 40, 59, 3) to chocolate (*e*.*g*. 37, 76, 85, 46 in CMYK color space). An exemplary gamut of "White" skin colors can range from light pinkish *(e.g.* 3, 8, 11, 0 in CMYK color space) to peachy pink *(e.g.* 6, 25, 24, 0 in CMYK color space). In the present disclosure, the term "human skin color" may be understood as comprising any color interpolated from the aforementioned coordinates in CMYK color space, *e.g*. within any one of the aforementioned exemplary gamuts or between any (two or more) of the aforementioned exemplary gamuts.

The first component may be any one of the plurality of components, *i.e.* a component selected from the group consisting of, the at least one base layer, the at least one interface member, the at least one resilient member, and the at least one cover layer. The second component may be any other one of the plurality of components, *i.e.* another component selected from the group consisting of, the at least one base layer, the at least one interface member, the at least one resilient member, and the at least one cover layer. The third component may be any yet another one of the plurality of components, *i.e.* yet another component selected from the group consisting of, the at least one base layer, the at least one interface member, the at least one resilient member, and the at least one cover layer.

The fourth component may be any yet yet another one of the plurality of components, *i.e.* yet yet another component selected from the group consisting of, the at least one base layer, the at least one interface member, the at least one resilient member, and the at least one cover layer.

The plurality of components may comprise a first set of components, wherein, for each component of the first set, a portion or an entirety of the first major surface of the respective component constitutes a corresponding portion of the first side / face of the nasal dilator (in use), *i.e.* of an outermost surface of the nasal dilator facing in the first direction (in use). The plurality of components may comprise a second set of components, wherein, for each component of the second set, the first major surface of the respective component is affixed - *e.g*. (adhesively) adhered, (ultrasonically, thermally, and/or adhesively) bonded, pressure bonded, or stitched - to the second major surface of at least one other component of the plurality of components and the second major surface of the respective component is affixed - *e.g*. (adhesively) adhered, (ultrasonically, thermally, and/or adhesively) bonded, pressure bonded, or stitched - to the first major surface of at least one other component of the plurality of components. In other words, each component of the second set may be sandwiched between other components of the plurality of components. The plurality of components may comprise a third set of components, wherein, for each component of the third set, a portion or an entirety of the second major surface of the respective component constitutes a corresponding portion of the second side / face of the nasal dilator (in use), *i.e.* of an outermost surface of the nasal dilator facing in the second direction (in use). The plurality of components may consist of the first, second and third sets.

Each (individual, respective component) of the plurality of components may comprise a (respective, individual) outer periphery. The (respective, individual) outer periphery may comprise at least one straight-line segment parallel to a longitudinal axis of the nasal dilator. The (respective, individual) outer periphery may comprise at least one segment corresponding to at least a portion of an (overall) outer periphery of the nasal dilator. The (respective, individual) outer periphery consisting of at least one segment selected from the group consisting of a straight line parallel to the longitudinal axis of the nasal dilator and a segment corresponding to at least a portion of an outer periphery of the nasal dilator. For example, an entire outer periphery of any component belonging to the plurality of components may correspond to an outer periphery of the nasal dilator. The outer periphery of any component belonging to the plurality of components may comprise / consist of two straight segments parallel to the longitudinal axis of the nasal dilator, a first end of the two straight segments being joined by a first segment corresponding to a segment of the outer periphery of the nasal dilator, and a second end of the two straight segments being joined by a second segment corresponding to another segment of the outer periphery of the nasal dilator.

The nasal dilator may have an overall shape that permits *n* nasal dilators of said overall shape to be die-cut from a sheet of laminated materials, wherein the sheet of laminated materials has an area less than *n* times an area of a minimally sized rectangular bounding box for said overall shape.

The nasal dilator may be shaped to conform to a(n average) user's nose and to (contactingly) cover both a portion of a left cheek less than 2 cm or less than 1 cm below a left eye socket and a portion of a right cheek less than 2 cm or less than 1 cm below a right eye socket. The average user may be selected from the group consisting of an adult, an adolescent, and a pre-adolescent. The nasal dilator may comprise / consist of a central portion, a left cheek portion, and a right cheek portion. More specifically, the nasal dilator may comprise a central portion configured to bridge a nose (of an adult, an adolescent, or a pre-adolescent), *e.g.* for the sake of dilating a nasal passage of the (wearer's) nose, a left cheek portion configured to extend across a portion of a maxilla (upper jaw bone) less than 2 cm or less than 1 cm below a left eye socket to a portion of a left zygoma (a bone at a lower, outer corner of an eye socket) less than 2 cm or less than 1 cm below a left eye socket, and a right cheek portion configured to extend across a portion of the maxilla less than 2 cm or less than 1 cm below a right eye socket to a portion of a right zygoma (a bone at a lower, outer corner of an eye socket) less than 2 cm or less than 1 cm below a right eye socket. The central portion may cover at least 60% or at least 80% of a(n average) user's nose. The left cheek portion may have an area of at least 1 cm², at least 1.5 cm², at least 2 cm², at least 2.5 cm², or at least 3 cm². The right cheek portion may have an area of at least 1 cm², at least 1.5 cm², at least 2 cm², at least 2.5 cm², or at least 3 cm². The left cheek portion may be larger than a rectangle having a length to width ratio of 2:1 and an area of at least 1 cm², at least 1.5 cm², at least 2 cm², at least 2.5 cm², or at least 3 cm². The right cheek portion may be larger than a rectangle having a length to width ratio of 2:1 and an area of at least 1 cm², at least 1.5 cm², at least 2 cm², at least 2.5 cm², or at least 3 cm². The left cheek portion may have an area of at least 1 cm², at least 1.5 cm², at least 2 cm², at least 2.5 cm², or at least 3 cm². The right cheek portion may have an area of at least 1 cm², at least 1.5 cm², at least 2 cm², at least 2.5 cm², or at least 3 cm². An entirety or at least 40%, at least 60%, or at least 80% of an entirety of an area (of a second major surface) of the left cheek portion may be of / coated with a material that absorbs at least 40%, at least 60%, or at least 80% of incident light (in the visible spectrum). An entirety or at least 40%, at least 60%, or at least 80% of an entirety of an area (of a second major surface) of the right cheek portion may be of / coated with a material that absorbs at least 40%, at least 60%, or at least 80% of incident light (in the visible spectrum). An entirety or at least 40%, at least 60%, or at least 80% of an entirety of an area (of a second major surface) of the central portion may be of / coated with a material that absorbs at least 40%, at least 60%, or at least 80% of incident light (in the visible spectrum). The light-absorbing material may be of a dark color. The dark color may be a color for which at least two, at least three, or all four of the four halftone percentage values representing the dark color in CMYK color space are (individually) larger than 50, larger than 60, or larger than 70. The dark color may be a color for which at least the K (key / black) component of the four halftone percentage values representing the dark color in CMYK color space is (individually) larger than 50, larger than 60, or larger than 70.

The resilient member may be of a natural material, a synthetic material, or a combination of natural and synthetic materials. For example, the resilient member may comprise a thermoplastic resin, *e.g*. a (biaxially oriented) polyester resin such as (biaxially oriented) poly(ethylene terephthalate), often referred to by the abbreviation boPET / PET. The resilient member may have a thickness (in a direction perpendicular to the first / second major surface of the resilient member) in the range of 0.05 mm to 1 mm, *e.g*. in the range of 0.1 mm to 0.5 mm. The resilient member may have a width of not less than 10%, not less than 20%, or not less than 30% of a minimum width of the nasal dilator (perpendicular to a longitudinal axis of the nasal dilator). The resilient member may have a width of not more than 30%, not more than 40%, or not more than 50% of a minimum width of the nasal dilator (perpendicular to a longitudinal axis of the nasal dilator). The resilient member may have a length (along a longitudinal axis of the nasal dilator coaxial to a longitudinal axis of the (respective) resilient member) of less than 90%, or less than 95% of a length of the nasal dilator (along a longitudinal axis of the nasal dilator coaxial to a longitudinal axis of the (respective) resilient member). Each longitudinal end of the resilient member may be distanced from an outermost peripheral edge of the nasal dilator, *e.g*. by at least 5% or at least 10% of a length of the nasal dilator (along a longitudinal axis of the nasal dilator coaxial to a longitudinal axis of the (respective) resilient member). The resilient member may have a length of not less than 80%, not less than 90%, or not less than 95% of a length of the nasal dilator (along a longitudinal axis of the nasal dilator coaxial to a longitudinal axis of the (respective) resilient member). The ends of the resilient member may coincide with respective edges of the nasal dilator. End regions of the resilient member may be forked. A longitudinal axis of any one of the at least one resilient member may be parallel or oblique (by no more than 5° or by no more than 10°) to a longitudinal axis of any other of the at least one resilient member. As touched upon above, each of the at least one resilient member may comprise a first major surface and a second major surface. Any of the at least one resilient member may exhibit rigidity and/or no (macroscopic) elasticity in response to (manual) forces parallel to the first / second major surface. Any of the at least one resilient member may be (manually) deformable. Any of the at least one resilient member may be (manually) deformable in response to (manual) forces perpendicular to the first / second major surface. Such deformation of the respective resilient member may comprise resiliently flexing the respective resilient member such that a longitudinal axis of the respective resilient member is bent. The at least one resilient member may be deformed as a result of affixing the nasal dilator to a nose, *i*.*e*. affixing the nasal dilator to a user's nostrils across a bridge of a (user's) nose. More specifically, the at least one resilient member may be deformed as a result of a (resilient) flexing of the nasal dilator over a bridge of a (user's) nose, *i.e.* as a result of a (resilient) flexing of the nasal dilator to at least partially conform to a contour of the bridge of the (user's) nose. The at least one resilient member may exert return forces in response to the deformation, *e.g*. return forces that, in the absence of other forces, would return the at least one resilient member to an undeformed / unbiased state. The return forces emanating from the at least one resilient member, *e.g*. in in response to a deformation of the at least one resilient member resulting from an affixing of the nasal dilator to a user's nostrils across a bridge of a nose, may (be sufficient to) dilate a nasal passage of the (user's) nose.

The base layer and/or the cover layer may be of a natural material, a synthetic material, or a combination of natural and synthetic materials. For example, the base layer and/or the cover layer may comprise a woven or nonwoven fabric and/or a plastic film, *e.g.* a flexible fabric / film that is (comfortably) conformable to human skin, *e.g*. to a human nose. The plastic film may be a medical grade and/or a biocompatible film. The plastic film may be a thermoplastic and/or polyurethane film. The plastic film may have a moisture vapor transmission rate of at least 200 grams, at least 400 grams, or at least 800 grams per square meter per 24 hours. The moisture vapor transmission rate may be determined by a standard test such as, for example, ASTM F1249, ASTM E96, or ASTM2622. The plastic film may be an elastic film. The plastic film may be capable of stretching elastically in response to a (dilating / stretching) force in the plane of the plastic film. The plastic film may inherently return to an initial, unbiased state in response to cessation of such a (dilating / stretching) force. A total area of the first major surface of a component belonging to the at least one base layer or to the at least one cover layer may be (in terms of a numeric value) at least 10% and less than 50%, less than 40% or less than 30% of a total area of the first outermost surface. Similarly, the total area of the first major surface of a component belonging to the at least one base layer or to the at least one cover layer may be (in terms of a numeric value) at least 60%, at least 80%, at least 90% of a total area of the first outermost surface. The outermost peripheral edge of the base layer and/or the cover layer may define / correspond to an entire outermost peripheral edge of the nasal dilator or at least 60%, at least 80%, or at least 90% of an entire outermost peripheral edge of the nasal dilator. Similarly, the outermost peripheral edge of the base layer and/or the cover layer may define / correspond to less than 50%, less than 40%, or less than 30% of an entire outermost peripheral edge of the nasal dilator.

The present disclosure furthermore discloses a nasal dilator manufacture method, *e.g.* a method for manufacturing at least a sub-component of a nasal dilator as disclosed *supra.*

The method may comprise, for each of a plurality of components, laminating a respective one (component) of the plurality of components to at least one other (component) of the plurality of components. The plurality of components may be a plurality of components as described above. Any of the components belonging to the plurality of components may be in an unfinished state. For example, any of the components may be laminated before being cut into final shape. For example, any of the laminated components may be part of a sheet or strip of material from which the respective, individual component may be formed (merely) by cutting the material along (a path corresponding to) the outer perimeter of the nasal dilator. Similarly, any of the laminated components may be part of a sheet or strip of material that comprises at least two such individual components, e.g. components that may be separated in a later cutting operation. The laminating may yield a laminate as described above, *i.e.* a laminate that constitutes a structure of a nasal dilator.

The method may comprise adhering a (sheet of material that forms at least one) release liner to the first outermost surface of the laminate. Similarly, the method may comprise adhering a (sheet of material that forms at least one) cover liner to the to the second outermost surface of the laminate.

The method may comprise cutting, *e.g*. die-cutting, the laminate (together with the release liner and/or cover liner (material)), *e.g*. to yield a plurality of nasal dilators or a plurality of structures of a nasal dilator.

The various embodiments of the present disclosure having been described above in general terms, the embodiments shown in the Figures will now be elucidated.

In the Figures, stippling is used to schematically depict colors that cannot otherwise be depicted in black-and-white drawings. In each figure, differing stippling patterns are used to schematically depict differing colors, *i*.*e*. differing monochromatic / polychromatic appearances. More specifically, in each figure, a plurality of differing stippling patterns is used to schematically depict a corresponding plurality of differing colors, *i.e.* a corresponding plurality of differing monochromatic / polychromatic appearances. Each respective individual stippling pattern may represent a respective individual color, *e.g.* a color as described above. For example, a respective individual stippling pattern may represent a (humanly, visually) perceptible (monochromatic) color, not excluding black, white, or any shade of gray. Similarly, a respective individual stippling pattern may represent a (humanly, visually) perceptible polychromatic appearance, not excluding (constituents of) black, white, and/or any shades of gray.

Figure 2A schematically depicts a nasal dilator 200 in accordance with the present disclosure, e.g. as described above.

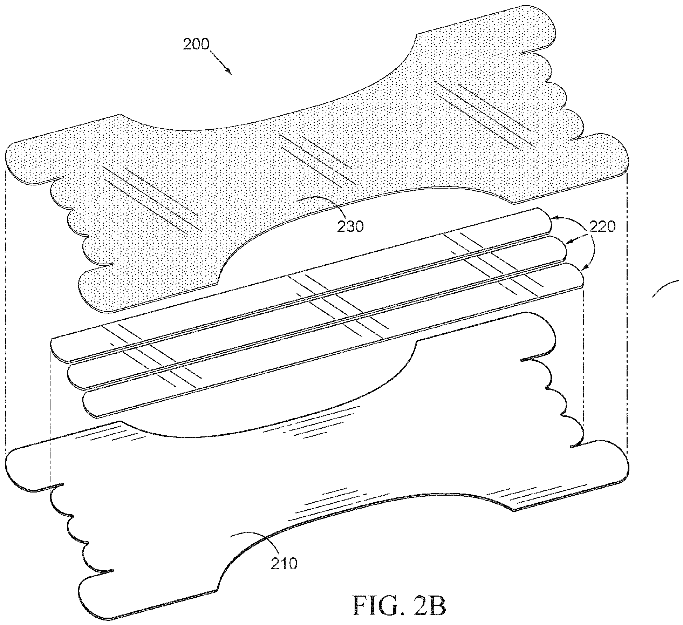

Figure 2B is an exploded view schematically depicting nasal dilator 200 of Fig. 2A. In the illustrated embodiment, nasal dilator 200 comprises a base layer 210, three resilient members 220A, 220B and 220C, and a semi-transparent cover layer 230. A second major surface of base layer 210 is of a first color; resilient members 220A, 220B and 220C may be opaque or non-opaque, *e.g*. opaque and of a second color; and semi-transparent cover layer 230 is colored with a third color. The first color may be bright white; the second color may be white; and the third color may be pink. The first color of base layer 210 may appear as a fourth color, *e.g.* a vibrant neon pink, when viewed through cover layer 230. The second color of resilient members 220A, 220B and 220C may appear as a fifth color when viewed through cover layer 230, which fifth color may appear less vibrant than and contrast with the fourth color. An entirety of an outer periphery of base layer 210 corresponds to an outer periphery of nasal dilator 200. An entirety of an outer periphery of cover layer 230 corresponds to an outer periphery of nasal dilator 200. An outer periphery of resilient member 220A consists of two straight segments parallel to a longitudinal axis of nasal dilator 200, a first end of the two straight segments being joined by a first segment corresponding to a segment of the outer periphery of nasal dilator 200, and a second end of the two straight segments being joined by a second segment corresponding to another segment of the outer periphery of nasal dilator 200. The same holds for an outer periphery of resilient member 220B as well as for an outer periphery of resilient member 220C.

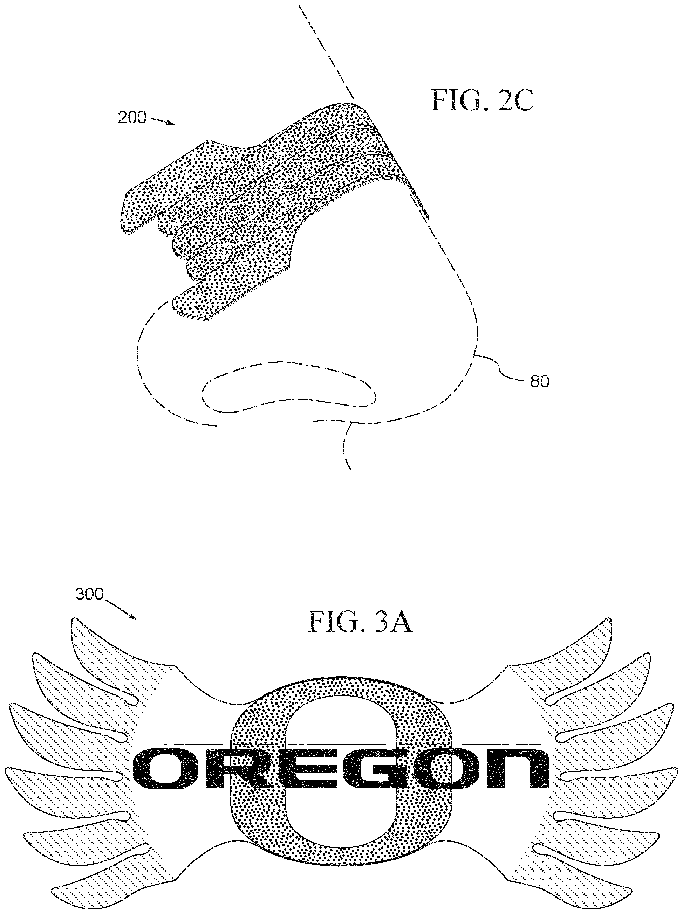

Figure 2C schematically depicts nasal dilator 200 of Fig. 2A in use, *i*.*e*. flexed over the bridge of and removably adhesively affixed to a user's nose 80.

Figure 3A schematically depicts a nasal dilator 300 in accordance with the present disclosure, *e.g*. as described above.

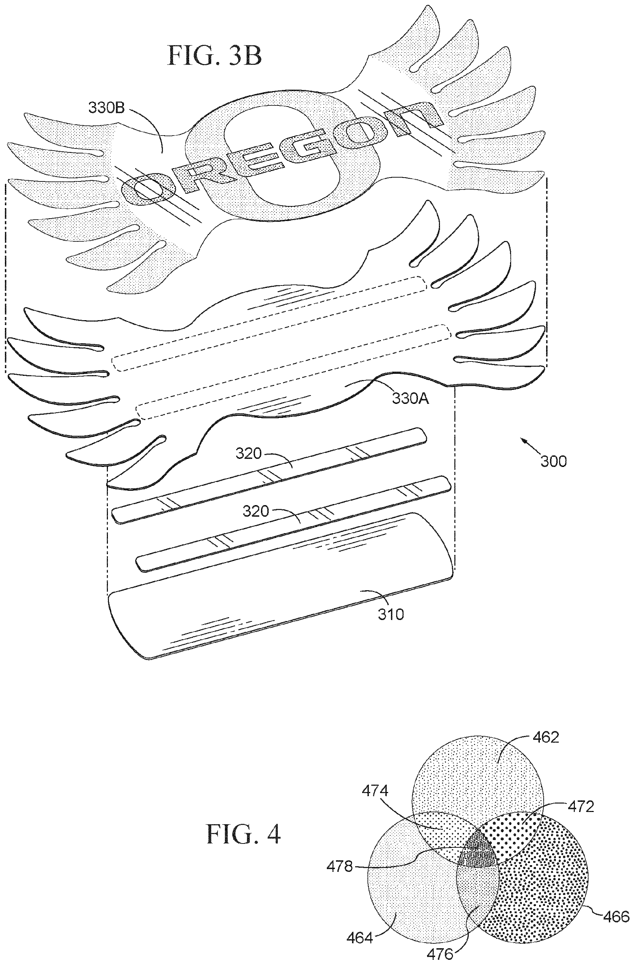

Figure 3B is an exploded view schematically depicting nasal dilator 300 of Fig. 3A. In the illustrated embodiment, nasal dilator 300 comprises a base layer 310, two resilient members 320, and two cover layers 330A and 330B. A second major surface of cover layer 330A is of a first color, and a second major surface of cover layer 330B comprises an opaque multi-color logo. Excepting a region occluded by the opaque multi-color logo, cover layer 330B is transparent. The transparency of cover layer 330B visibly exposes a portion of the second major surface of cover layer 330A. The first color may be bright white. The letters in the word "OREGON" in the multi-color logo may be colored dark green with a yellow outline that matches a yellow coloring of the large letter "O" in the multi-color logo. The large letter "O" in the multi-color logo may be colored yellow with a dark green outline that matches the dark green letters of the word "OREGON". A contour of resilient members 320 is recognizable through cover member 330A and frames the word "OREGON" in the multi-color logo.

Figure 4 schematically depicts a model of subtractive color mixing. In the depiction, the stippling in region 462 represents the primary color red; the stippling in region 464 represents the primary color yellow; the stippling in region 466 represents the primary color blue; the stippling in region 472 represents purple, *i*.*e*. a subtractive mixing of the primary colors red and blue; the stippling in region 474 represents orange, *i.e.* a subtractive mixing of the primary colors red and yellow; the stippling in region 476 represents green, *i*.*e*. a subtractive mixing of the primary colors yellow and blue; and the stippling in region 478 represents black, *i.e.* a subtractive mixing of the primary colors red, yellow and blue.

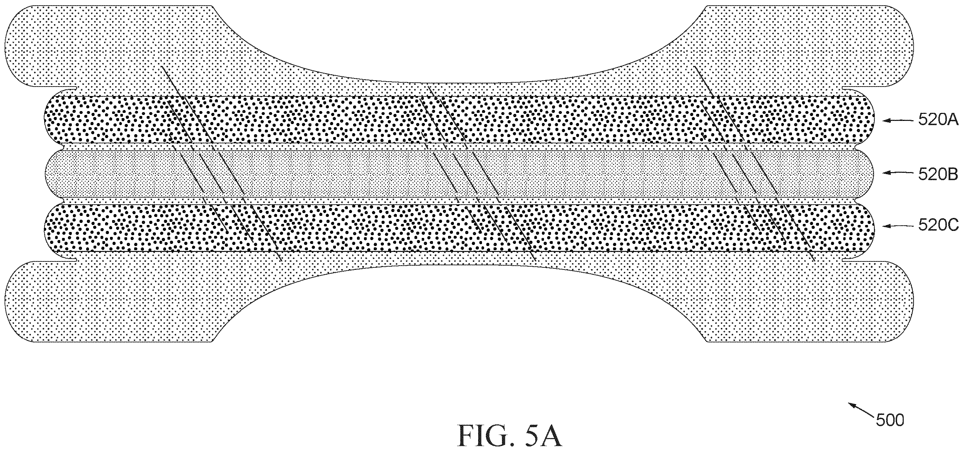

Figure 5A schematically depicts a nasal dilator 500 in accordance with the present disclosure, *e.g.* as described above.

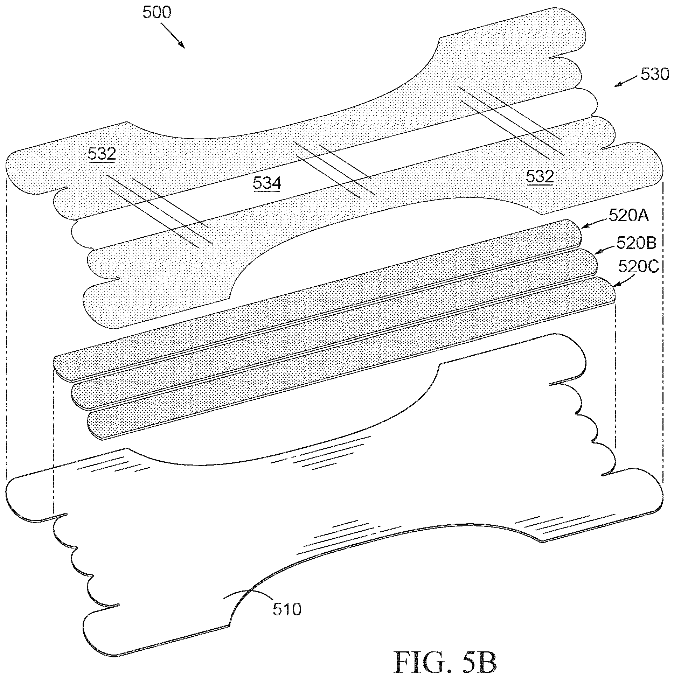

Figure 5B is an exploded view schematically depicting nasal dilator 500 of Fig. 5A. In the illustrated embodiment, nasal dilator 500 comprises a base layer 510, three resilient members 520A, 520B and 520C, and a cover layer 530. A second major surface of base layer 510 is of a first color; resilient members 520A, 520B, 520C may be opaque or non-opaque, *e.g*. opaque and of a second color. Cover layer 530 comprises two semi-transparent regions 532 of a third color and a transparent region 534. The first color of base layer 510 may appear as a fourth color, when viewed through cover layer 530. The second color of resilient members 520A and 520C may appear as a fifth color when viewed through the third color of semi-transparent regions 532 of cover layer 530. The second color of resilient member 520B will appear unaltered when viewed through transparent region 534 of cover layer 530. The first color may be bright white; the second color may be blue; the third color may be pale yellow; the fourth color may be vibrant yellow; and the fifth color may be bright green.

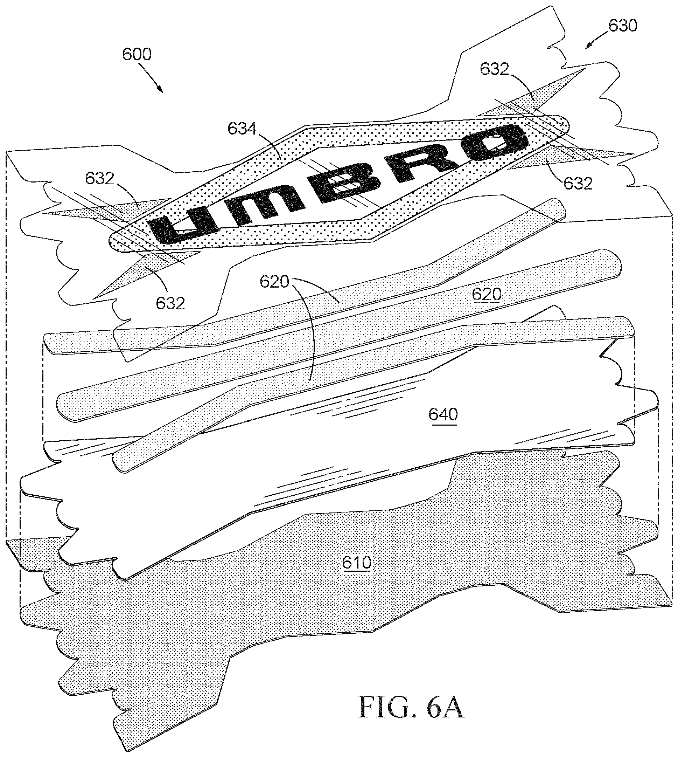

Figure 6A is an exploded view schematically depicting a nasal dilator 600, *e.g.* as described above. In the illustrated embodiment, nasal dilator 600 comprises a base layer 610, three resilient members 620, a cover layer 630, and an interface member 640. A second major surface of base layer 610 is of a first color; a second major surface of interface member 640 is of a second color; and a second major surface of resilient members 620 is of a third color. A second major surface of cover layer 630 comprises a semi-transparent multi-color logo. Excepting a region occupied by by the multi-color logo, cover layer 630 is transparent. The multi-color logo comprises four semi-transparent regions 632 of a fourth color and a semi-transparent region 634 of a fifth color. The first color may be black; the second color may be white; the third color may be yellow; the fourth color may be orange; and the fifth color may be red. The letters in the word "UMBRO" in the multi-color logo may also be of the first color, *i.e.* black, and may be outlined with the fourth color, *i.e.* orange. Each of regions 632 may comprise a border of the fifth color, *i.e.* bright red.

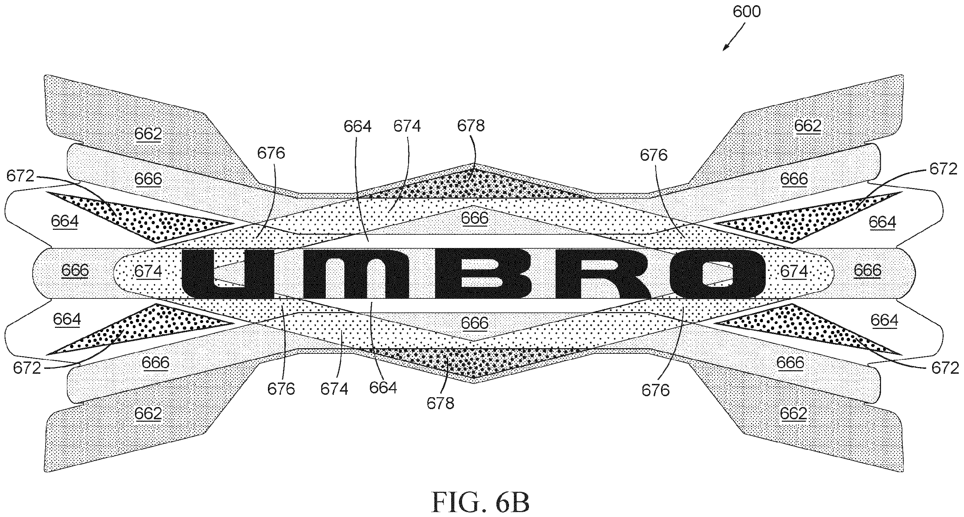

Figure 6B schematically depicts nasal dilator 600 of Figure 6A in an assembled state. In the depiction, the stippling in regions 662 represents the first color, *i.e.* regions where the transparency of cover layer 630 visibly exposes a portion of the second major surface of base layer 610. The absence of stippling in regions 664 represents the second color, *i.e.* regions where the transparency of cover layer 630 visibly exposes a portion of the second major surface of interface member 640. The stippling in regions 666 represents the third color, *i*.*e*. regions where the transparency of cover layer 630 visibly exposes a portion of the second major surface of resilient members 620. The stippling in regions 672 represents a sixth color, *i*.*e*. regions where the second color of interface member 640 is visible via semi-transparent regions 632 and the fourth color of semi-transparent regions 632 alters a visual perception of the second color of interface member 640, the second color appearing via the fourth color as the sixth color, e.g. bright orange. The stippling in regions 674 represents a seventh color, *i*.*e*. regions where the third color of resilient members 620 is visible via semi-transparent region 634 and the fifth color of semi-transparent region 634 alters a visual perception of the third color of resilient members 620, the third color appearing via the fifth color as the seventh color, *e.g.* orange. The stippling in regions 676 represents an eighth color, *i*.*e*. regions where the second color of interface member 640 is via semi-transparent region 634 and the fifth color of semi-transparent region 634 alters a visual perception of the second color of interface member 640, the second color appearing via the fifth color as the eighth color, *e.g.* bright red. The stippling in regions 678 represents a ninth color, *i.e.* regions where the first color of base layer 610is via semi-transparent region 634 and the fifth color of semi-transparent region 634 alters a visual perception of the first color of base layer 610, the first color appearing via the fifth color as the ninth color, *e.g*. dark red.

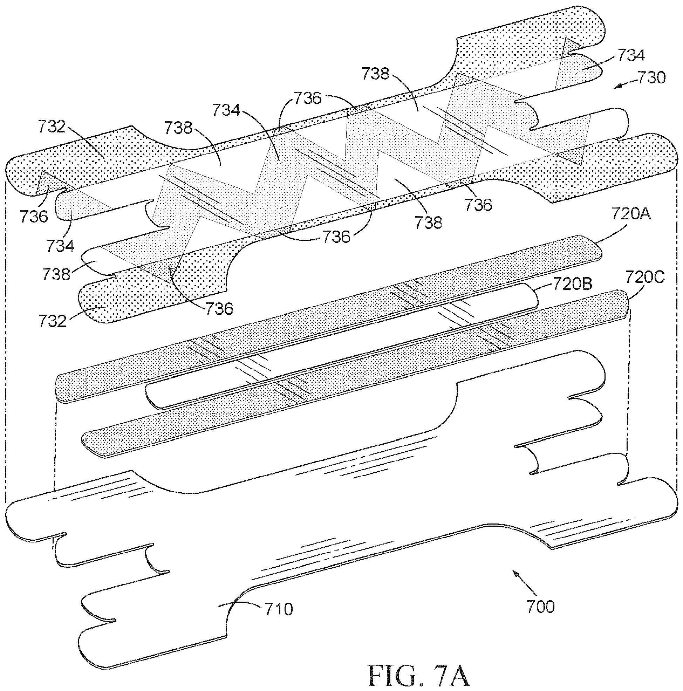

Figure 7A is an exploded view schematically depicting a nasal dilator 700, *e.g*. as described above. In the illustrated embodiment, nasal dilator 700 comprises a base layer 710, three resilient members 720A, 720B and 720C, and a cover layer 730. A second major surface of base layer 710 is of a first color; a second major surface of resilient members 720A and 720C is of a second color; and resilient member 720B is vastly translucent, *i.e.* nearly transparent. A second major surface of cover layer 730 comprises a semi-transparent multi-color ornamentation. The multi-color ornamentation comprises semi-transparent regions 732 of a third color, semi-transparent regions 734 of a fourth color, and semi-transparent regions 736 of a fifth color. A remainder of cover layer 730 is transparent, as exemplified by regions 738. The first color may be bright white; the second color may be red; the third color may be blue; the fourth color may be light gray; and the fifth color may be pale blue.

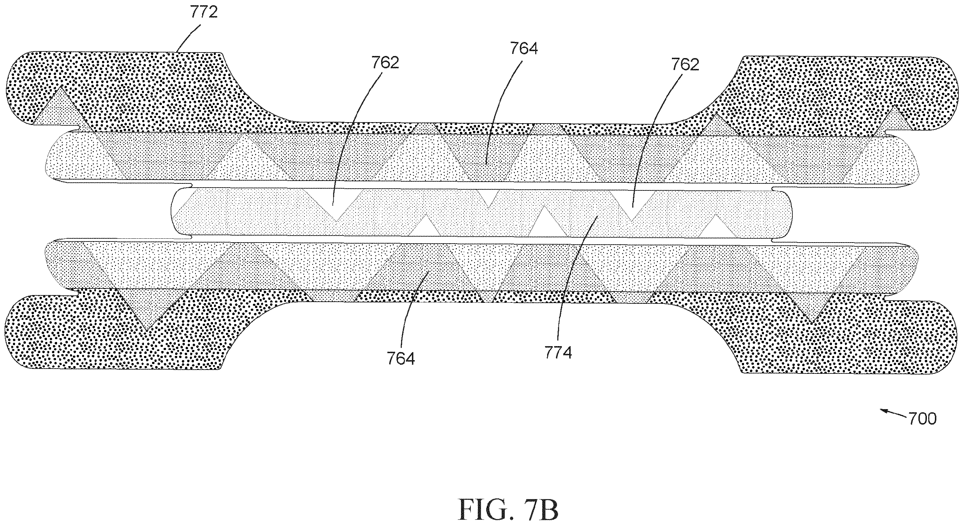

Figure 7B schematically depicts nasal dilator 700 of Figure 7A in an assembled state. In the depiction, the various types of stippling represent the various colors produced by the mixing / interaction of the colors / translucency of base layer 710 / resilient members 720A, 720B, 720C with the colors / transparency of cover layer 730. For example, the absence of stippling in regions 762 represents the third color, *i*.*e*. regions where the transparency of cover layer 730 and the vast translucency of resilient member 720B allow the first color of the second major surface of base layer 710 to shine through essentially uninhibited. Similarly, the stippling in regions 764 represents the second color, *i.e.* regions where the transparency of cover layer 730 visibly exposes a portion of the second major surface of resilient members 720A, 720C. The stippling in regions 772 represents a sixth color, *i*.*e*. regions where the first color of base layer 710 is visible via semi-transparent regions 732 and the third color of semi-transparent regions 732 alters a visual perception of the first color of base layer 710, the first color appearing via the third color as the sixth color, *e.g*. bright blue. The stippling in regions 774 represents a seventh color, *i.e.* regions where resilient member 720B - by virtue of its vast translucency / near transparency - does not significantly obscure the first color of the second major surface of base layer 710 and the fourth color of semi-transparent regions 634 consequently interacts with light passing toward / from base layer 710, the fourth color of semi-transparent regions 634 thus (predominantly) altering a visual perception of the first color of base layer 710, the first color appearing via the fourth color as the seventh color, *e.g.* very light gray.

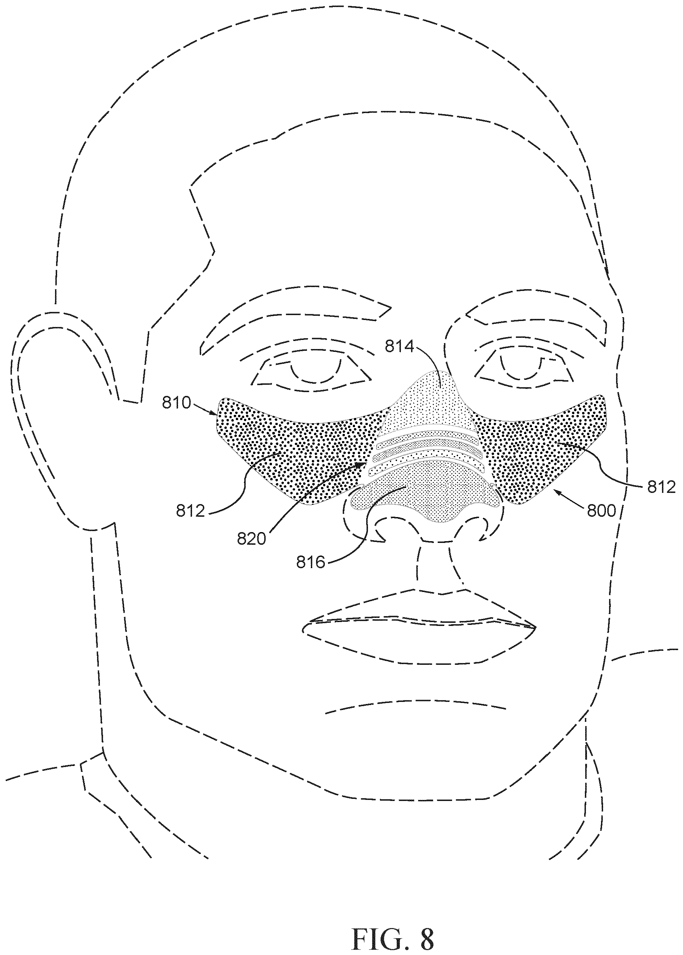

Figure 8 schematically depicts a nasal dilator 800 in accordance with the present disclosure, *e.g.* as described above. In the illustrated embodiment, nasal dilator 800 comprises a base layer 810 and three resilient members 820. Base layer 810 comprises a central portion and two cheek portions 812, *viz.* a left cheek portion and a right cheek portion. A first region 814 of central portion is of a first (dark) color, and a second region 816 of central portion is of a second (dark) color. Each of the two cheek portions 812 is of a third (dark) color. Each of resilient members 820 is semi-transparent and of a respective color. The first color of the fist region 814 of central portion may be visually perceived as another (fourth / fifth / sixth) color, when viewed through a respective color of a respective one of semi-transparent resilient members 820.

In the present disclosure, the verb "may" is used to designate optionality / noncompulsoriness. In other words, something that "may" can, but need not. In the present disclosure, the verb "comprise" may be understood in the sense of including. Accordingly, the verb "comprise" does not exclude the presence of other elements / actions. In the present disclosure, relational terms such as "first," "second," "top," "bottom" and the like may be used solely to distinguish one entity or action from another entity or action without necessarily requiring or implying any actual such relationship or order between such entities or actions.

In the present disclosure, the term "any" may be understood as designating any number of the respective elements, *e.g.* as designating one, at least one, at least two, each or all of the respective elements. Similarly, the term "any" may be understood as designating any collection(s) of the respective elements, *e.g*. as designating one or more collections of the respective elements, wherein a (respective) collection may comprise one, at least one, at least two, each or all of the respective elements. The respective collections need not comprise the same number of elements.

In the present disclosure, the expression "at least one" is used to designate any (integer) number or range of (integer) numbers (that is technically reasonable in the given context). As such, the expression "at least one" may, *inter alia,* be understood as one, two, three, four, five, ten, fifteen, twenty or one hundred. Similarly, the expression "at least one" may, *inter alia,* be understood as "one or more," "two or more" or "five or more."

In the present disclosure, expressions in parentheses may be understood as being optional. As used in the present disclosure, quotation marks may emphasize that the expression in quotation marks may also be understood in a figurative sense. As used in the present disclosure, quotation marks may identify a particular expression under discussion.

In the present disclosure, many features are described as being optional, e.g. through the use of the verb "may" or the use of parentheses. For the sake of brevity and legibility, the present disclosure does not explicitly recite each and every combination and/or permutation that may be obtained by choosing from the set of optional features. However, the present disclosure is to be interpreted as explicitly disclosing all such combinations / permutations. For example, a system described as having three optional features may be embodied in seven different ways, namely with just one of the three possible features, with any two of the three possible features or with all three of the three possible features.

While various embodiments of the present invention have been disclosed and described in detail herein, it will be apparent to those skilled in the art that various changes may be made to the configuration, operation and form of the invention without departing from the spirit and scope thereof. In particular, it is noted that the respective features of the invention, even those disclosed solely in combination with other features of the invention, may be combined in any configuration excepting those readily apparent to the person skilled in the art as nonsensical. Likewise, use of the singular and plural is solely for the sake of illustration and is not to be interpreted as limiting. Except where the contrary is explicitly noted, the plural may be replaced by the singular and vice-versa.

The above disclosure may be summarized as comprising the following embodiments.

### Embodiment 1:

A nasal dilator, comprising:
a plurality of laminated components, wherein
each of said laminated components comprises a first major surface that faces in a first direction and a second major surface that faces in a second direction,
said plurality of laminated components comprises a first component and a second component,
said first component comprises a first region of a first color,
said second component comprises a second region,
said second region is a semi-transparent region of a second color, and
said second region overlaps said first region such that said first color is visible via said second region and said second color alters a visual perception of said first color.

### Embodiment 2:

The nasal dilator of Embodiment 1, wherein:
said first region comprises an entirety of said second major surface of said first component.

### Embodiment 3:

The nasal dilator of Embodiment 1 or 2, wherein:
said second region comprises an entirety of said second component.

### Embodiment 4:

The nasal dilator of Embodiment 1 or 2, wherein:
said second component comprises a third region, and
said third region is a semi-transparent region of a third color, and
said third region overlaps said first region such that said first color is visible via said third region and said third color alters a visual perception of said first color.

### Embodiment 5:

The nasal dilator of Embodiment 4, wherein:
a first sum of four halftone percentage values representing said first color in CMYK color space differs from a second sum of four halftone percentage values representing said second color in CMYK color space by at least a first given minimum, said first given minimum being selected from the group consisting of 5, 8, and 10, and
a third sum of four halftone percentage values representing said third color in CMYK color space differs from each of said first sum and said second sum by at least a second given minimum, said second given minimum being selected from the group consisting of 5, 8, and 10.

### Embodiment 6:

The nasal dilator of any one of Embodiments 1, 2, and 4, wherein:
said second component comprises a fourth region, and
said fourth region is an opaque region of a fourth color.

### Embodiment 7:

The nasal dilator of Embodiment 6, wherein:
a fourth sum of four halftone percentage values representing said first color in CMYK color space differs from an fifth sum of four halftone percentage values representing said second color in CMYK color space by at least a third given minimum, said third given minimum being selected from the group consisting of 5, 8, and 10, and
a sixth sum of four halftone percentage values representing said fourth color in CMYK color space differs from each of said fourth sum and said fifth sum by at least a fourth given minimum, said fourth given minimum being selected from the group consisting of 5, 8, and 10.

### Embodiment 8:

The nasal dilator of any one of Embodiments 1, 2, and 4-7, wherein:
said second component comprises a fifth region, and
said fifth region is transparent.

### Embodiment 9:

The nasal dilator of any one of Embodiments 1-8, wherein:
said plurality of laminated components comprises at least one resilient member,
said second component is a first cover layer,
said first component is selected from the group consisting of an optional first base layer, an optional second base layer, an optional third base layer, a first resilient member of said at least one resilient member, an optional second resilient member of said at least one resilient member, an optional third resilient member of said at least one resilient member, an optional second cover layer, and an optional third cover layer.

### Embodiment 10:

The nasal dilator of any one of Embodiments 1-8, wherein:
said plurality of laminated components comprises at least one resilient member,
said second component is a first resilient member of said at least one resilient member, and
said first component is selected from the group consisting of an optional first base layer, an optional second base layer, an optional third base layer, an optional second resilient member of said at least one resilient member, an optional third resilient member of said at least one resilient member, an optional first cover layer, an optional second cover layer, and an optional third cover layer.

### Embodiment 11:

The nasal dilator of Embodiment 9 or 10, wherein:
said second major surface of said first base layer, if present, faces said first major surface of at least one respective resilient member of said at least one resilient member,
said second major surface of said second base layer, if present, faces said first major surface of at least one respective resilient member of said at least one resilient member,
said second major surface of said third base layer, if present, faces said first major surface of at least one respective resilient member of said at least one resilient member,
said first major surface of said first cover layer, if present, faces said second major surface of at least one respective resilient member of said at least one resilient member,
said first major surface of said second cover layer, if present, faces said second major surface of at least one respective resilient member of said at least one resilient member, and
said first major surface of said third cover layer, if present, faces said second major surface of at least one respective resilient member of said at least one resilient member.

### Embodiment 12:

The nasal dilator of any one of Embodiments 9-11, wherein:
return forces emanating from said at least one resilient member in response to a deformation of said at least one resilient member resulting from an affixing of said nasal dilator to a user's nostrils across a bridge of a nose dilate a nasal passage of said nose.

### Embodiment 13:

The nasal dilator of any one of Embodiments 9-12, wherein:
said plurality of laminated components comprises a third component,
an entirety of said second major surface of said third component is of a fifth color,
said second region overlaps said second major surface of said third component such that said fifth color is visible via said second region and said second color alters a visual perception of said fifth color, and
said third component is another component selected from the group consisting of said first base layer, said second base layer, said third base layer, said first resilient member, said second resilient member, said third resilient member, said first cover layer, said second cover layer, and said third cover layer.

### Embodiment 14:

The nasal dilator of any one of Embodiments 1-12, wherein:
said plurality of laminated components comprises a third component,
an entirety of said second major surface of said third component is of a fifth color, and
said second region overlaps said second major surface of said third component such that said fifth color is visible via said second region and said second color alters a visual perception of said fifth color.

### Embodiment 15:

The nasal dilator of Embodiment 13 or 14, wherein:
a seventh sum of four halftone percentage values representing said first color in CMYK color space differs from an eighth sum of four halftone percentage values representing said second color in CMYK color space by at least a fifth given minimum, said fifth given minimum being selected from the group consisting of 5, 8, and 10, and
a ninth sum of four halftone percentage values representing said fifth color in CMYK color space differs from each of said seventh sum and said eighth sum by at least a sixth given minimum, said sixth given minimum being selected from the group consisting of 5, 8, and 10.

### Embodiment 16:

The nasal dilator of any one of Embodiments 1-4, 6, and 8-14, wherein:
a tenth sum of four halftone percentage values representing said first color in CMYK color space differs from an eleventh sum of four halftone percentage values representing said second color in CMYK color space by at least a seventh given minimum, said seventh given minimum being selected from the group consisting of 5, 8, and 10.

### Embodiment 17:

The nasal dilator of any one of Embodiments 1-16, wherein:
a twelfth sum of four halftone percentage values representing said first color in CMYK color space differs from a thirteenth sum of four halftone percentage values representing a first human skin color closest to said first color in CMYK color space by at least an eighth given minimum, said eighth given minimum being selected from the group consisting of 5, 8, and 10, and
a fourteenth sum of four halftone percentage values representing said second color in CMYK color space differs from a fifteenth sum of four halftone percentage values representing a second human skin color closest to said second color in CMYK color space by at least a ninth given minimum, said ninth given minimum being selected from the group consisting of 5, 8, and 10.

### Embodiment 18:

The nasal dilator of any one of Embodiments 1-17, comprising:
at least one substance selected from the group consisting of a thermochromic substance and a photochromic substance, wherein
at least one of said first color and said second color is a transient color, and
said at least one substance contributes to said transient color.

### Embodiment 19:

A nasal dilator manufacture method, comprising:
for each of a plurality of components, laminating a respective one of said plurality of components to at least one other of said plurality of components, and
die-cutting said plurality of laminated components, wherein
said die-cut plurality of laminated components constitutes a structure of a nasal dilator,
each of said die-cut plurality of laminated components comprises a first major surface that faces in a first direction and a second major surface that faces in a second direction,
said die-cut plurality of laminated components comprises a first component and a second component,
said first component comprises a first region of a first color,
said second component comprises a second region,
said second region is a semi-transparent region of a second color, and
said second region overlaps said first region such that said first color is visible via said second region and said second color alters a visual perception of said first color.

### Embodiment 20:

The method of Embodiment 19, wherein:
said die-cut plurality of laminated components comprises a third component,
an entirety of said second major surface of said third component is of a third color, and
said second region overlaps said second major surface of said third component such that said third color is visible via said second region and said second color alters a visual perception of said third color.

## Claims

1. A nasal dilator, comprising:
a plurality of laminated components, wherein
each of said laminated components comprises a first major surface that faces in a first direction and a second major surface that faces in a second direction,
said plurality of laminated components comprises a first component and a second component,
said first component comprises a first region of a first color,
said second component comprises a second region,
said second region is a semi-transparent region of a second color, and
said second region overlaps said first region such that said first color is visible via said second region and said second color alters a visual perception of said first color.

2. The nasal dilator of claim 1, wherein:
said first region comprises an entirety of said second major surface of said first component.

3. The nasal dilator of claim 1 or 2, wherein:
said second region comprises an entirety of said second component.

4. The nasal dilator of claim 1 or 2, wherein:
said second component comprises a third region, and
said third region is a semi-transparent region of a third color, and
said third region overlaps said first region such that said first color is visible via said third region and said third color alters a visual perception of said first color.

5. The nasal dilator of claim 4, wherein:
a first sum of four halftone percentage values representing said first color in CMYK color space differs from a second sum of four halftone percentage values representing said second color in CMYK color space by at least a first given minimum, said first given minimum being selected from the group consisting of 5, 8, and 10, and
a third sum of four halftone percentage values representing said third color in CMYK color space differs from each of said first sum and said second sum by at least a second given minimum, said second given minimum being selected from the group consisting of 5, 8, and 10.

6. The nasal dilator of any one of claims 1, 2, and 4, wherein:
said second component comprises a fourth region, and
said fourth region is an opaque region of a fourth color.

7. The nasal dilator of claim 6, wherein:
a fourth sum of four halftone percentage values representing said first color in CMYK color space differs from an fifth sum of four halftone percentage values representing said second color in CMYK color space by at least a third given minimum, said third given minimum being selected from the group consisting of 5, 8, and 10, and
a sixth sum of four halftone percentage values representing said fourth color in CMYK color space differs from each of said fourth sum and said fifth sum by at least a fourth given minimum, said fourth given minimum being selected from the group consisting of 5, 8, and 10.

8. The nasal dilator of any one of the preceding claims, wherein:
said plurality of laminated components comprises at least one resilient member,
said second component is a first cover layer,
said first component is selected from the group consisting of an optional first base layer, an optional second base layer, an optional third base layer, a first resilient member of said at least one resilient member, an optional second resilient member of said at least one resilient member, an optional third resilient member of said at least one resilient member, an optional second cover layer, and an optional third cover layer.

9. The nasal dilator of any one of claims 1-7, wherein:
said plurality of laminated components comprises at least one resilient member,
said second component is a first resilient member of said at least one resilient member, and
said first component is selected from the group consisting of an optional first base layer, an optional second base layer, an optional third base layer, an optional second resilient member of said at least one resilient member, an optional third resilient member of said at least one resilient member, an optional first cover layer, an optional second cover layer, and an optional third cover layer.

10. The nasal dilator of any one of claims 8-9, wherein:
said plurality of laminated components comprises a third component,
an entirety of said second major surface of said third component is of a fifth color,
said second region overlaps said second major surface of said third component such that said fifth color is visible via said second region and said second color alters a visual perception of said fifth color, and
said third component is another component selected from the group consisting of said first base layer, said second base layer, said third base layer, said first resilient member, said second resilient member, said third resilient member, said first cover layer, said second cover layer, and said third cover layer.

11. The nasal dilator of any one of claims 1-9, wherein:
said plurality of laminated components comprises a third component,
an entirety of said second major surface of said third component is of a fifth color, and
said second region overlaps said second major surface of said third component such that said fifth color is visible via said second region and said second color alters a visual perception of said fifth color.

12. The nasal dilator of any one of the preceding claims, wherein:
a seventh sum of four halftone percentage values representing said first color in CMYK color space differs from an eighth sum of four halftone percentage values representing a first human skin color closest to said first color in CMYK color space by at least a fifth given minimum, said fifth given minimum being selected from the group consisting of 5, 8, and 10, and
a ninth sum of four halftone percentage values representing said second color in CMYK color space differs from a tenth sum of four halftone percentage values representing a second human skin color closest to said second color in CMYK color space by at least a sixth given minimum, said sixth given minimum being selected from the group consisting of 5, 8, and 10.

13. The nasal dilator of any one of the preceding claims, comprising:
at least one substance selected from the group consisting of a thermochromic substance and a photochromic substance, wherein
at least one of said first color and said second color is a transient color, and
said at least one substance contributes to said transient color.

14. A nasal dilator manufacture method, comprising:
for each of a plurality of components, laminating a respective one of said plurality of components to at least one other of said plurality of components, and
die-cutting said plurality of laminated components, wherein
said die-cut plurality of laminated components constitutes a structure of a nasal dilator,
each of said die-cut plurality of laminated components comprises a first major surface that faces in a first direction and a second major surface that faces in a second direction,
said die-cut plurality of laminated components comprises a first component and a second component,
said first component comprises a first region of a first color,
said second component comprises a second region,
said second region is a semi-transparent region of a second color, and
said second region overlaps said first region such that said first color is visible via said second region and said second color alters a visual perception of said first color.

15. The method of claim 14, wherein:
said die-cut plurality of laminated components comprises a third component,
an entirety of said second major surface of said third component is of a third color, and
said second region overlaps said second major surface of said third component such that said third color is visible via said second region and said second color alters a visual perception of said third color.
